# EUROPEAN PATENT APPLICATION

(11) **EP 1 731 183 A1**
(43) Date of publication of application: **13.12.2006**
(21) Application number: 05727567.9
(22) Date of filing: 30.03.2005
(51) Int. Cl.: A61M 1/14, A61K 9/16, A61K 31/191, A61K 31/7004, A61K 33/06, A61K 33/14, A61P 7/08

(54) **SOLID PHARMACEUTICAL PREPARATION FOR DIALYSIS**

(30) Priority: 30.03.2004 JP 2004097287; 06.04.2004 JP 2004111674; 13.05.2004 JP 2004144223
(71) Applicant: NIPRO CORPORATION, Osaka-shi, Osaka 531-8510 (JP)
(72) Inventor: KAI, Toshiya, c/o NIPRO CORPORATION, Osaka-shi, Osaka 5318510 (JP); KATAYAMA, Naohisa, c/o NIPRO CORPORATION, Osaka-shi, Osaka 5318510 (JP); YOKOE, Junichi, c/o NIPRO CORPORATION, Osaka-shi, Osaka 5318510 (JP); SATO, Makoto, c/o NIPRO CORPORATION, Osaka-shi, Osaka 5318510 (JP)
(74) Representative: Albrecht, Thomas
(86) International application number: PCT/JP2005/006092
(87) International publication number: WO 2005/094918

(57) **Abstract**

An object of the present invention is to provide a solid pharmaceutical preparation, in which the solid pharmaceutical preparation does not contain any acetic acid, the other electrolyte components are substantially the same as that of conventional preparation for dialysis as well as the method for preparing the dialysate, and which is excellent in content uniformity and storage stability, and a production process thereof. The inventors of the present invention have studied earnestly to solve the problems and finally found out that the problems can be solved by a solid pharmaceutical preparation for dialysis containing two pharmaceutical preparations: a solid pharmaceutical preparation (A) composed of particles having an average particle size of about 100 µm to 1, 500 µm and containing one or more electrolytes selected from the group consisting of sodium chloride, calcium chloride, magnesium chloride, and potassium chloride, and an organic acid other than acetic acid and/or a salt of the organic acid; and a solid pharmaceutical preparation (B) containing sodium bicarbonate, thereby achieving the present invention.

## Description

### Technical Field

The present invention relates to a solid pharmaceutical preparation for dialysis containing electrolyte components and a pH-adjusting agent, which is provided for preparing a bicarbonate-containing dialysate to be used for blood purification treatment such as hemodialysis.

### Background Art

Among the most common treatment for blood purification is hemodialysis (i.e., artificial dialysis) which is carried out on patients with chronic renal failure or the like. Hemodialysis intends to improve serum electrolytic concentration, correct acid-base equilibrium, and the like, in addition to removing waste products and water. A bicarbonate ion, which is an alkaline agent in the living body, is a small molecule, so it can be removed by dialysis. For preventing severe hypobicarbonatemia from occurring, a large amount of an alkaline agent is required in a dialysate to be used. Thus, it is natural that bicarbonate is an optimal alkaline agent. However, the bicarbonate ions react with calcium ions and magnesium ions to generate insoluble compounds (i.e., metal carbonates such as calcium carbonate and magnesium carbonate). Thus, bicarbonate ions are unstable in a bicarbonate dialysate and bacteria tend to proliferate therein, thereby causing problems such as difficulty in long-period storage and so on.

An attempt to supply an alkali agent has been established by utilizing a mechanism that an acetic acid is metabolized in the liver to convert into bicarbonate, thereby allowing a stable dialysate to be supplied. Therefore, an acetate dialysate using acetate as an alkaline agent has come into use. As a result, it has become possible to set a high alkaline concentration and supply sufficient bicarbonate. However, acetic acid may cause inhibitory effects on cardiac functions as well as angiectasia. For acetate intolerance patients having slow acetate metabolism, problems such as respiratory depression have been caused by deterioration of dialysis disequilibrium syndrome and depletion of a large amount of CO₂ in blood under dialysis, which are attributable to acetic acid.

Thereafter, because of an increase in dialysis patients and an expansion of the disease to be treated by dialysis, such as diabetes, the frequency and severity of the dialysis disequilibrium syndrome have increased. In addition, demands on asymptomatic dialysis, which induces slight uncomfortableness during the dialysis even in the case of normal dialysis patients, have been increased. Furthermore, the participation of acetic acid as a cause of these symptoms has been strongly suspected. Currently, therefore, the mainstream of dialysis has shifted from acetate dialysis using acetate as an alkaline agent to bicarbonate dialysis using sodium hydrogencarbonate.

Generally, in bicarbonate dialysis, a dialysate is comprised of two pharmaceutical preparations: "preparation A" which is a solid pharmaceutical preparation containing electrolytic components including calcium ions and magnesium ions, a pH-adjusting agent, and/or glucose; and "preparation B" which is a solid pharmaceutical preparation containing sodium hydrogencarbonate serving as a bicarbonate ion. This composition is provided for avoiding the generation of metal carbonate which is an insoluble compound, which is due to the reaction of bicarbonate ions with calcium ions and magnesium ions.

In addition, the composition of a pharmaceutical preparation for dialysis containing sodium bicarbonate, which is currently available in the market, is represented below. In spite of being referred to as a preparation for sodiumbicarbonate dialysis, the preparation still contains acetic acid, sodium acetate, or the like as a pH-adjusting agent, specifically, 2 to 12 mEq/L of acetic acid under present circumstances, even though the contents thereof are smaller than that of the former acetate dialysis.
- Na⁺: 120-150 mEq/L
- K⁺: 0.5-3.0 mEq/L
- Ca⁺: 1.5-4.5 mEq/L
- Mg⁺: 0-2.0 mEq/L
- Cl⁻: 90-135 mEq/L
- HCO₃⁻: 20-35 mEq/L
- CH₃COO⁻: 2.0-12 mEq/L
- Glucose: 0-2.5 g/L

At first, it was considered that the addition of acetic acid or sodium acetate of about 2 to 12 mEq/L as a pH-adjusting agent would be of no consequence. However, recently, with prolongation of dialysis, the emergence of clinical manifestations such as headache and hypotension during dialysis, which may be caused by acetic acid, have become a problem because acetic acid is scarcely present in the living body (equal to or less than 0.1 mEq/L) in nature. In addition, the performance improvement of a dialyzer or the like has an adverse effect on a circulatory organ because of an excessive load of acetic acid. Therefore, the fact that the toxicity of acetic acid, such as acetate intolerance, is stronger than expected has come to be recognized.

Technologies related to pharmaceutical preparations using citric acid and sodium citrate to prevent the precipitation of calcium carbonate or magnesium carbonate in use have been conventionally known (Patent Document 1). However, no formulation related to a pharmaceutical preparation which does not contain acetic acid (hereinafter, referred to as "acetate-free preparation") has been disclosed. In addition, even though some technologies related to an acetate-free preparation have been already published (Patent Documents 2 and 3), preparations having uniform components and excellent storage stability (i.e., prolonged storage stability in particular) have not been disclosed. In addition, for manufacturing a single-preparation type pharmaceutical preparation for dialysis, in which sodium bicarbonate and electrolytes are housed in a chamber, a production process using citric acid as a solid organic acid has been disclosed (Patent Documents 3 to 7), although the production process concurrently uses sodium acetate.
Therefore, there has been a demand for developing a solid pharmaceutical preparation for dialysis which completely removes acetic acid, and which can be used under physiological conditions as is the case with conventional preparation.

Patent Document 1: JP 10-87478 A
Patent Document 2: JP 2003-339853 A
Patent Document 3: JP 2003-104869 A
Patent Document 4: JP 06-335527 A
Patent Document 5: JP 06-335528 A
Patent Document 6: JP 08-092070 A
Patent Document 7: JP 08-092071 A
Patent Document 8: JP 08-169836 A

### Disclosure of the Invention

### Problems to be solved by the Invention

An object of the present invention is to provide a solid pharmaceutical preparation, in which the solid pharmaceutical preparation does not contain any acetic acid, other electrolyte components are substantially the same as that of the conventional preparations for dialysis as well as the method for preparing the dialysate, and which is excellent in content uniformity and storage stability, and a production process thereof.

### Means for solving the Problems

The inventors of the present invention have studied earnestly to solve the above-mentioned problems and finally discovered that the above-mentioned problems can be solved by a solid pharmaceutical preparation for dialysis which contains two pharmaceutical preparations: a solid pharmaceutical preparation (A) composed of particles having an average particle size of about 100 µm to 1,500 µm and containing one or more electrolytes selected from the group consisting of sodium chloride, calcium chloride, magnesium chloride, and potassium chloride; and an organic acid other than acetic acid and/or a salt of the organic acid; and a solid pharmaceutical preparation (B) containing sodium bicarbonate, thereby achieving the present invention.

That is, the present invention relates to:
(1) a solid pharmaceutical preparation for dialysis, comprising the following two solid pharmaceutical preparations:
   a solid pharmaceutical preparation (A) having an average particle size of about 100 µm to 1,500 µm and containing one or more electrolytes selected from the group consisting of sodium chloride; calcium chloride, magnesium chloride, and potassium chloride and an organic acid other than acetic acid and/or a salt of the organic acid; and
   a solid pharmaceutical preparation (B) containing sodium bicarbonate;
(2) the solid pharmaceutical preparation for dialysis according to item 1, wherein the organic acid other than the acetic acid is at least one organic acid selected from the group consisting of citric acid, oxalic acid, tartaric acid, maleic acid, ascorbic acid, oxaloacetic acid, gluconic acid, isocitric acid, malic acid, and pyruvic acid;
(3) the solid pharmaceutical preparation for dialysis according to item 1, wherein:
   the solid pharmaceutical preparation (A) comprises particles each including core particle containing sodium chloride covered with a coating layer containing at least one electrolyte selected from the group consisting of: calcium chloride, magnesium chloride, and potassium chloride, a plurality of the particles being bound to each other;
(4) a process for producing the solid pharmaceutical preparation for dialysis according to item 1, comprising the steps:

(1) spraying an aqueous solution containing at least one electrolyte selected from the group consisting of calcium chloride, magnesium chloride, and potassium chloride onto core particles containing sodium chloride, and then drying the core particles; and
(2) mixing the particles obtained by the step (1) with particles containing citric acid and/or a salt thereof, thereby producing a solid pharmaceutical preparation (A);
(5) a process for producing the solid pharmaceutical preparation for dialysis according to item 1, comprising the steps of spraying an aqueous solution containing at least one electrolyte selected from the group consisting of calcium chloride, magnesium chloride, and potassium chloride, and an organic acid other than acetic acid and/or a salt of the organic acid onto core particles containing sodium chloride, and then drying the core particles, thereby producing a solid pharmaceutical preparation (A);
(6) the solid pharmaceutical preparation for dialysis according to item 1, wherein the solid pharmaceutical preparation (A) comprises:
   first particles containing one or more electrolytes selected from the group consisting of sodium chloride, calcium chloride, magnesium chloride, and potassium chloride, the electrolytes being uniformly distributed within the particles; and
   second particles containing an organic acid other than acetic acid and/or a salt of the organic acid;
(7) the solid pharmaceutical preparation for dialysis according to item 1, wherein the solid pharmaceutical preparation (A) comprises particles containing one or more electrolytes selected from the group consisting of sodium chloride, calcium chloride, magnesium chloride, and potassium chloride, and an organic acid other than acetic acid and/or a salt of the organic acid, the electrolytes and the organic acid other than acetic acid and/or the salt thereof being uniformly distributed within the particles.
(8) a process for producing the solid pharmaceutical preparation for dialysis according to item 1, comprising the steps of:
   (1) spraying and drying an aqueous solution containing at least one electrolyte selected from the group consisting of sodium chloride, calcium chloride, magnesium chloride, and potassium chloride to thereby obtain granulated substances (granules) ; and
   (2) mixing the granulated substances with particles containing citric acid and/or a salt thereof, thereby producing a solid pharmaceutical preparation (A); and
(9) a process for producing the solid pharmaceutical preparation for dialysis according to item 1, comprising the step of spraying and drying an aqueous solution containing at least one electrolyte selected from the group consisting of sodium chloride, calcium chloride, magnesium chloride, and potassium chloride, and an organic acid other than acetic acid and/or a salt of the organic acid, thereby producing a solid pharmaceutical preparation (A).

### Effects of the Invention

The characteristic feature of the solid pharmaceutical preparation for dialysis of the present invention is the use of an organic acid (e.g., citric acid) and/or an organic salt (e.g., sodium citrate) that can be found in the living body, while acetic acid and acetate are not used at all. Therefore, an acetate-free sodium bicarbonate dialysate can be prepared.
The conventional solid pharmaceutical preparation for dialysis contains acetic acid and/or acetate. In contrast, the solid pharmaceutical preparation for dialysis of the present invention does not contain acetic acid or acetate at all, but contains sodium hydrogencarbonate as a main alkaline agent. Therefore, the preparation of the present invention is a more physiologically acceptable formulation, so that the preparation of the present invention can prevent dialysis patients (particularly, acetate intolerance patients) and the like from suffering from any adverse effect caused by acetic acid. In addition, the solid pharmaceutical preparation for dialysis of the present invention has good content uniformity and excellent storage stability, so a sufficient amount thereof can be stored in medical facilities where dialysis therapies are carried out.

Furthermore, in the solid pharmaceutical preparation for dialysis of the present invention, the granulated substances in the solid pharmaceutical preparation (A), which are manufactured by a spray-drying granulation process, are the granulated substances in which electrolytes are uniformly distributed and no variation is found in solubility. In addition, as the organic acid and/or the salt thereof, a solid organic acid and/or a salt thereof is/are used, so that the strength of the granulated substance can be raised increased and an effect of preventing the generation of fine particles during transportation or storage can be obtained.

Furthermore, the solid pharmaceutical preparation for dialysis of the present invention is characterized in that the solid pharmaceutical preparation does not use acetic acid and acetate at all, but uses a solid organic acid and/or a salt thereof which are/is found in the living body. Therefore, an acetate-free sodium bicarbonate dialysate can be prepared.
Furthermore, the solid pharmaceutical preparation for dialysis of the present invention can be prepared by substituting for sodium acetate conventionally contained in an amount of 2.0 mEq/mL to 12 mEq/mL, an equal amount of an organic salt, and also substituting a solid organic acid for acetic acid, thereby resulting in a pharmaceutical preparation for dialysis excellent in safety without causing precipitation during preparation, which has the same concentrations of electrolytes (i.e., sodium ion, potassium ion, magnesium ion, calcium ion, and bicarbonate ion) as those of the conventional pharmaceutical preparations for dialysis.

### Best Modes for carrying out the Invention

The solid pharmaceutical preparation for dialysis of the present invention is a solid pharmaceutical preparation to be dissolved in water to prepare a sodium bicarbonate-containing dialysate. The solid pharmaceutical preparation contains a solid pharmaceutical preparation (A) and a solid pharmaceutical preparation (B) described below.
The solid pharmaceutical preparation (A) is comprised of particles having an average particle size of about 100 µm to 1,500 µm, which contains one or more electrolytes selected from the group consisting of sodium chloride, calcium chloride, magnesium chloride, and potassium chloride and an organic acid other than acetic acid and/or a salt of the organic acid.
The solid pharmaceutical preparation (B) is a solid pharmaceutical preparation containing sodium bicarbonate.

In the present invention, the solid pharmaceutical preparation (A) contains one or more electrolytes selected from the group consisting of sodium chloride, calcium chloride, magnesium chloride, and potassium chloride and an organic acid other than acetic acid and/or a salt of the organic acid serving as a pH-adjusting agent. Preferably, the solid pharmaceutical preparation (A) may contain sodium chloride, potassium chloride, calcium chloride, and magnesium chloride. Furthermore, the solid pharmaceutical preparation (A) may additionally contain glucose.

The average particle size of particles in the solid pharmaceutical preparation (A) corresponds to the average particle size of all particles in the solid pharmaceutical preparation (A) that contains the above-mentioned particles and other particles, and is preferably in the range of about 100 µm to 1, 500 µm. In view of content uniformity, the average particle size is more preferably in the range of about 100 to 800 µm, most preferably in the range of about 180 to 800 µm.
If several kinds of particles (including particles) are contained in the solid pharmaceutical preparation (A), it is preferable that these particles be uniformly mixed.

The particles used for the present invention may include granulated substances obtained by various known granulation processes, combinations thereof, and particles of simple powders. Preferably, those particles may be uniformly mixed in the solid pharmaceutical preparation (A) and at least one kind of particles in the solid pharmaceutical preparation (A) may be uniformly mixed.

If at least one kind of particle contains granulated substances, it is preferable that a core particle containing sodium chloride of the granulated substance be a particle coated with a coating layer containing at least one electrolyte selected from the group consisting of calcium chloride, magnesium chloride, and potassium chloride.
The core particle containing sodium chloride is a particle made of a compound containing sodium chloride. The core particle may be any particle as far as it contains sodium chloride. For example, the core particle may be a particle containing merely sodium chloride or containing an organic acid other than acetic acid (e.g., citric acid) and/or a salt thereof (e.g., sodium citrate) as well as sodium chloride.
The average particle size of the core particle is not particularly limited, but generally is in the range of about 15 µm to 70 µm.

The coating layer containing one or more electrolytes selected from the group consisting of calcium chloride, magnesium chloride, and potassium chloride is a layer that substantially coats the core particle, but there is no need of completely coating the core particle. The coating layer may contain, in addition to the respective components described above, an organic acid other than acetic acid (e.g., citric acid) and/or a salt thereof (e.g., sodium citrate). Preferably, furthermore, the coating layer may have the content uniformity of electrolytes therein.

In the present invention, it is preferable that the above-mentioned particles are bound to each other in combinations of plural particles. The configuration of the combination of plural particles may be prepared such that particles, in which the surfaces of all of the core particles alone are coated with coating layers, are generated first and the particles are then bound to each other through the coating layers. Alternatively, it may be prepared such that the core particles are attached with each other first and the coating layers are then formed over the particles, or the particles bound together are further bound to each other through the coating layers. Furthermore, though the coating layer is formed on the surface of the core particle, there is a possibility that some particles remain alone uncoated In any case, similar effects can be obtained in formation of a solid pharmaceutical preparation for dialysis having the uniformity of components and excellent storage stability, both of which are the object of the present invention.

An example of preferable composition of electrolytes in the present invention is a composition containing sodium chloride, potassium chloride, magnesium chloride, calcium chloride, sodium hydrogencarbonate, and citric acid. The composition may be further mixed with glucose and/or sodium citrate. The blending amount of each electrolyte in the combination can be suitably determined to fall in the range represented in the table below when it is diluted into an appropriate concentration for a dialysate.
- Na⁺: 120-160 mEq/L
- K⁺: 0.5-3.0 mEq/L
- Ca⁺: 1.5-4.5 mEq/L
- Mg⁺: 0-2.0 mEq/L
- Cl⁻: 90-135 mEq/L
- HCO₃⁻: 20-35 mEq/L
- Organic acid ion: 2.0-20 mEq/L
- Glucose: 0-2.5 g/L

Organic acids, other than acetic acid, to be used in the present invention are preferably solid organic acids, and of the solid organic acids, one or more organic acids selected from the group consisting of citric acid, oxalic acid, tartaric acid, maleic acid, ascorbic acid, oxaloacetic acid, gluconic acid, isocitric acid, malic acid, and pyruvic acid are preferable. Of these, citric acid is particularly preferable.
The solid pharmaceutical preparation for dialysis of the present invention may preferably contain an organic acid in an amount sufficient to adjust the pH of a dialysate to about 7.0 to 7.6 when it is prepared as a dialysate.
When citric acid is used as the organic acid, the solid pharmaceutical preparation for dialysis of the present invention may preferably contain citric acid in an amount required to adjust the pH of a dialysate to about 7.2 to 7.4 in use. The content of the citric acid is suitably determined depending on the amount of sodium bicarbonate in the solid pharmaceutical preparation (B).
The solid pharmaceutical preparation for dialysis of the present invention may preferably contain citric acid and/or a salt thereof in an amount sufficient to adjust the citrate ion concentration in the dialysate to 2 to 20 mEq/mL in general when it is prepared as a dialysate.

Salts of organic acids except acetic acid are preferably solid organic acid salts, and of those one or more organic salts selected from the group consisting of sodium citrate, potassium citrate, calcium citrate, sodium dihydrogen citrate, disodium citrate, gluconic acid, calcium gluconate, sodium gluconate, potassium gluconate, magnesium gluconate, sodium pyruvate, and potassium pyruvate are preferable. Of these, sodium citrate is particularly preferable.
When sodium citrate is used as a salt of organic acid other than acetic acid, sodium citrate may be added in an amount required for adjusting pH to about 7.2 to 7.4 in use. In this case, the amount of sodium citrate depends on the amount of sodium bicarbonate in the solid pharmaceutical preparation (B).

The solid pharmaceutical preparation (B) of the present invention is not particularly limited as far as it is a solid pharmaceutical preparation containing sodiumbicarbonate. However, it is preferable that the amount of the sodium bicarbonate be in the range of about 120 to 160 mEq when it is diluted to a suitable concentration for a dialysate. The solid pharmaceutical preparation (B) may contain an additional organic salt other than acetic acid. In addition, the solid pharmaceutical preparation (B) may contain glucose.
Furthermore, the solid pharmaceutical preparation for dialysis of the present invention may be composed of the solid pharmaceutical preparation (A), the solid pharmaceutical preparation (B), and an additional solid pharmaceutical preparation containing glucose.

A process for producing the solid pharmaceutical preparation for dialysis of the present invention may employ any of various known general granulation processes, for example agitation granulation processes such as a fluidized-bed granulation process, a rolling fluidized-bed granulation process, and a double-can agitation granulation process and wet granulation processes such as an extrusion granulation process. Of these, the fluidized-bed granulation process is preferably used, and the rolling fluidized-bed granulation process is more preferably used.

According to the production process of the present invention, the solid pharmaceutical preparation (A) may be preferably produced by the step (1) of spraying an aqueous solution containing at least one electrolyte selected from the group consisting of calcium chloride, magnesium chloride, and potassium chloride on core particles containing sodium chloride and then drying, and the step (2) of mixing the particles obtained by the step (1) with particles containing citric acid and/or a salt thereof. The particles containing citric acid and/or a salt thereof may include not only those passed through a granulation process but also simple powders of citric acid and/or a salt thereof, or the like.
The above-mentioned core particles and/or the above-mentioned aqueous solution may each contain citric acid and/or a salt thereof.
Furthermore, in the above-mentioned step (2) or after the above-mentioned step (2), an additional step of mixing glucose may be included.

In another production process of the present invention, the solid pharmaceutical preparation (A) may be preferably prepared by the step of spraying an aqueous solution containing calcium chloride, magnesium chloride, potassium chloride, and citric acid on core particles containing sodium chloride and then drying. In the aqueous solution, citrate may be included. Furthermore, subsequent to this step, an additional step of mixing glucose may be included.

The above-mentioned core particle to be used in the production process of the present invention may contain citric acid and/or a salt thereof. In addition, other than containing the above-mentioned components, the aqueous solution may be an aqueous solution prepared by addition of an organic acid (e.g., citric acid) other than acetic acid and/or a salt of organic acid other than acetic acid (e.g., sodium citrate).

As another embodiment of the present invention, the solid pharmaceutical preparation (A) contains
(i) first particles containing one or more electrolytes selected from the group consisting of sodium chloride, calcium chloride, magnesium chloride, and potassium chloride, the electrolytes being uniformly distributed within the particles; and second particles containing an organic acid other than acetic acid and/or a salt of the organic acid, or
(ii) particles containing one or more electrolytes selected from the group consisting of sodium chloride, calcium chloride, magnesium chloride, and potassium chloride, as well as an organic acid other than acetic acid and/or a salt of the organic acid, the electrolytes and the organic acid other than acetic acid and/or a salt of the organic acid being uniformly distributed within the particles.

The phrase "the electrolytes are uniformly distributed within the particles" or "the electrolytes and an organic acid other than acetic acid and/or a salt of the organic acid are uniformly distributed within the particles" means that, every time part of the particles is randomly collected and the component composition thereof is then measured, the component composition is always constant regardless of the collected portions.
The above-mentioned particles may contain an organic acid (e.g., citric acid) other than acetic acid and/or a salt of the organic acid (e.g., sodium citrate) in addition to the electrolytic components. Furthermore, the above-mentioned particles may additionally contain glucose.

A process for producing the solid pharmaceutical preparation for dialysis of the present invention may employ various known spray-drying granulation processes used for the production of solid pharmaceutical preparations for dialysis.
In the present invention, an apparatus used for the spray-drying granulation process may be any of general apparatuses for spray-drying, or those capable of carrying out the formation of a fluidized layer such as fluidized layer granulation or rolling fluidized-layer granulation and spray drying. A spray-drying apparatus capable of carrying out spray-drying efficiently is preferably used. In the process of spray-drying granulation for a solid pharmaceutical preparation for sodium bicarbonate dialysis, an organic acid (e.g., citric acid) other than acetic acid and/or an organic salt other than acetate (e.g., sodium citrate) may be dissolved in an aqueous electrolyte solution tobe sprayed. Further, glucose may be concurrently dissolved to carry out spray-drying. The glucose has a possibility of being colored in general wet granulation owing to an influence of moisture and heat. However, according to the spray-drying of the present invention, it is characterized in that particles can be instantaneously granulated and dried, followed by being discharged to the outside of the apparatus, so that such coloring hardly occurs. In addition, the glucose may be added at the end or a suitable amount thereof may be added at the time of preparing a dialysate.

In the production process of the present invention, the solid pharmaceutical preparation (A) may be preferably produced by the step (1) of obtaining first particles by subjecting an aqueous solution containing at least one electrolyte selected from the group consisting of sodium chloride, calcium chloride, magnesium chloride, and potassium chloride, and the step (2) of mixing second particles containing citric acid and/or a salt thereof into the particles.
The second particles containing citric acid and/or a salt thereof include not only those passed through the granulation process but also include sole powders of citric acid and/or a salt thereof, or the like.
Other than containing one or more of the above-mentioned respective components, the above-mentioned aqueous solution may be one prepared by addition of an organic acid (preferably, citric acid) other than acetic acid and/or a salt of the organic acid (preferably, sodium citrate).
Furthermore, glucose may be dissolved in the above-mentioned aqueous solution. Alternatively, in the above-mentioned step (2) or after the above-mentioned step (2), glucose may be mixed into the above-mentioned aqueous solution.

In the manufacturing process of the present invention, the diameter of a droplet to be sprayed can be adjusted to be small by increasing the concentration of the aqueous solution in which electrolytes are dissolved, increasing the spraying rate of a spraying device, or reducing the air pressure of spray from the spraying device. In contrast, the diameter of a droplet to be sprayed may be adjusted to be large by reducing the concentration of the aqueous electrolyte solution, reducing the spraying rate of a spraying device, or increasing the air pressure of spray from the spraying device.
If the aqueous electrolyte solution to be sprayed is excessively diluted, it takes much time to carry out spray-drying, while an excessively high concentration thereof tends to cause coarse particles.

Solid organic acids such as citric acid and/or salts of the organic acid exert effects of enhancing the strength of spray-dried particles and suppressing the generation of fine particles during transportation and storage. Thus, an aqueous solution in which the solid organic acid and/or a salt thereof are/is dissolved together with other electrolytes may be preferably used to carry out spray-drying granulation. In addition, after the spray-drying granulation of the electrolyte solution, even if these powders are mixed together, it is possible to formulate a solid pharmaceutical preparation for dialysis having uniformity and excellent stability.

The solid pharmaceutical preparation for dialysis of the present invention is generally used by being dissolved in water for dialysis at a concentration of about 0.8 to 1.5% by weight based on the dialysate.

Hereinafter, the present invention will be described in detail with reference to examples. Note that, the present invention is not restricted by the examples described below.

### Example 1

An aqueous solution was prepared by completely dissolving 24.4 parts by weight of potassium chloride, 30.1 parts by weight of calcium chloride, 16.6 parts by weight of magnesium chloride, and 83.9 parts by weight of citric acid in 253.9 parts by weight of purified water. The above-mentioned aqueous solution was sprayed and dried onto 1, 000 parts by weight of sodium chloride having an average particle size of 300 µm, which was flowing in a rolling fluidized-bed granulator (MP-01, manufactured by Powrex, Co., Ltd.), under conditions of an intake-air temperature of 80°C and a rotor speed of 300 rpm, thereby obtaining granular particles having an average particle size of 500 µm. The granular particles were packaged in aluminum container, thereby providing a solid pharmaceutical preparation (A).
Furthermore, with respect to 1, 155 parts by weight of the solid pharmaceutical preparation (A), 385 parts by weight of sodium hydrogencarbonate and 164 parts by weight of glucose were separately packaged in aluminum container and then provided as a solid pharmaceutical preparation (B) and a glucose solid pharmaceutical preparation, respectively, thereby obtaining a solid pharmaceutical preparation for dialysis.

### Example 2

An aqueous solution was prepared by completely dissolving 24.4 parts by weight of potassium chloride, 30.1 parts by weight of calcium chloride, 16.6 parts by weight of magnesium chloride, and 26.2 parts by weight of citric acid in 196.1 parts by weight of purified water. The above-mentioned aqueous solution was sprayed and dried onto a mixture of powders of 1,000 parts by weight of sodium chloride and 88.3 parts by weight of sodium citrate having an average particle size of 300 µm, which were flowing in a rolling fluidized-bed granulator (MP-01, manufactured by Powrex, Co., Ltd.), under conditions of an intake-air temperature of 80°C and a rotor speed of 300 rpm, thereby obtaining granular particles having an average particle size of 500 µm. The granular particles were packaged in aluminum container, thereby providing a solid pharmaceutical preparation (A).
Furthermore, with respect to 1, 186 parts by weight of the solid pharmaceutical preparation (A), 385 parts by weight of sodium hydrogencarbonate and 164 parts by weight of glucose were separately packaged in and then provided as a solid pharmaceutical preparation (B) and a glucose solid pharmaceutical preparation, respectively, thereby obtaining a solid pharmaceutical preparation for dialysis.

### Example 3

An aqueous solution was prepared by completely dissolving 24.4 parts by weight of potassium chloride, 30.1 parts by weight of calcium chloride, 16.6 parts by weight of magnesium chloride, 88.3 parts by weight of sodium citrate, and 83.9 parts by weight of citric acid in 2, 400 parts by weight of purified water. The above-mentioned aqueous solution was sprayed and dried onto 1, 000 parts by weight of sodium chloride having an average particle size of 300 µm, whichwas flowing ina rolling fluidized-bedgranulator (MP-01, manufactured by Powrex, Co., Ltd.), under conditions of an intake-air temperature of 80°C and a rotor speed of 300 rpm, thereby obtaining granular particles having an average particle size of 500 µm. The granular particles were packaged in aluminum container, thereby providing a solid pharmaceutical preparation (A).

### Example 4

An aqueous solution was prepared by completely dissolving 24.4 parts by weight of potassium chloride, 30.1 parts by weight of calcium chloride, 16.6 parts by weight of magnesium chloride, and 88.3 parts by weight of sodium citrate in 1, 750 parts by weight of purified water. Then, the above-mentioned aqueous solution was sprayed and dried onto a mixture of powders of 1,000 parts by weight of sodium chloride and citric acid each having an average particle size of 300 µm, which were flowing in a rolling fluidized-bed granulator(MP-01, manufactured by Powrex,Co.,Ltd.), under conditions of an intake-air temperature of 80°C and a rotor speed of 300 rpm, thereby obtaining granular particles having an average particle size of 500 µm. The granular particles were packaged in aluminum container, thereby providing a solid pharmaceutical preparation (A).

### Example 5

As is the case with Example 1, sodium chloride core particles were coated with potassium chloride, calcium chloride, magnesium chloride, and citric acid, thereby obtaining granular particles (about 500 µm in average particle size).
In addition, 1,000 g of a 25 w/w% aqueous glucose solution was prepared. 1,000 parts by weight of glucose powders having an average particle size of 180 micrometer were placed in a rolling fluidized-bed granulator (MP-01, manufactured by Powrex, Co., Ltd.), and the glucose particles which were made to flow were then sprayed with 500 g of the aqueous glucose solution under conditions of an intake-air temperature of 60°C and a rotor speed of 300 rpm, thereby obtaining glucose particles having an average particle size of about 450 µm. 315 parts by weight of the glucose particles were added to 2, 221. 9 parts by weight of the above-mentioned granular particles, and then mixed in a V-type mixer to be packaged in aluminum container, thereby providing a solid pharmaceutical preparation (A).

### Example 6

An aqueous solution was prepared by completely dissolving 24.4 parts by weight of potassium chloride, 30.1 parts by weight of calcium chloride, 16.6 parts by weight of magnesium chloride, and 26.2 parts by weight of citric acid in 253.9 parts by weight of purified water. The above-mentioned aqueous solution was sprayed and dried onto 1, 000 parts by weight of sodium chloride having an average particle size of about 300 µm, which was flowing in a rolling fluidized-bed granulator (MP-01, manufactured byPowrex, Co., Ltd.), under conditions of an intake-air temperature of 80°C and a rotor speed of 300 rpm, thereby obtaining granular particles of an average particle size of about 500 µm. The granular particles were packaged in aluminum container, thereby providing a solid pharmaceutical preparation (A).
In a V-type mixer (V20, manufactured by Tokuju Seisakusyo), 385 parts by weight of sodium hydrogencarbonate was mixed with 88.3 parts of sodium citrate, thereby obtaining another particle composition. The particle composition was packaged in aluminum container, thereby providing a solid pharmaceutical preparation (B) .

### Example 7

An aqueous solution was prepared by completely dissolving 24.4 parts by weight of potassium chloride, 30.1 parts by weight of calcium chloride, and 16. 6 parts by weight of magnesium chloride in 170 parts by weight of purified water. Then, the above-mentioned aqueous solution was sprayed and dried onto mixture powders of 1,000 parts by weight of sodium chloride and 83.9 parts by weight of sodium citrate each having an average particle size of about 300 µm, which were flowing in a rolling fluidized-bed granulator (MP-01, manufactured by Powrex, Co., Ltd.), under conditions of an intake-air temperature of 80°C and a rotor speed of 300 rpm, thereby obtaining granular particles having an average particle size of 500 µm. The granular particles were packaged in aluminum container, thereby providing a solid pharmaceutical preparation (A).
Furthermore, with respect to 1, 111 parts by weight of the solid pharmaceutical preparation (A), 470 parts by weight of sodium hydrogencarbonate and 164 parts by weight of glucose were separately packaged in aluminum container and then provided as a solid pharmaceutical preparation (B) and a glucose solid pharmaceutical preparation, respectively, thereby obtaining a solid pharmaceutical preparation for dialysis.

### Example 8

A solid pharmaceutical preparation (A) was obtained by the same method as that of Example 1.
An aqueous solution was prepared by dissolving 88.3 parts by weight of sodium citrate in 50 parts by weight of water. The above-mentioned aqueous solution was sprayed and dried onto 385 parts by weight of sodium hydrogencarbonate flowing in a rolling fluidized-bed granulator (MP-01, manufactured by Powrex, Co., Ltd.) under conditions of an intake-air temperature of 60°C and a rotor speed of 300 rpm, thereby obtaining granular particles having an average particle size of about 250 µm and then packaged in aluminum container, thereby providing a solid pharmaceutical preparation (B).

### (Test Example 1)

From the solid pharmaceutical preparations (A) of the respective samples obtained in Examples 1 to 8 as described above, 10 g thereof were arbitrarily collected three times and each of them was then dissolved in water to prepare 50 mL of an aqueous solution. The contents of the respective components in the solution weremeasured. Table 1 shows the ratio (%) of eachmean of the measured contents to the theoretical value, and its CV (%) (i.e., variation coefficient).
Note that, sodium and potassium were each measured by using a flame photometer, calcium and magnesium were each measured by using ion chromatography, citric acid was measured by using HPLC-UV, chlorine was measured by a silver nitrate titration method, and glucose was measured by using a polarimeter. Table 1 shows the measurement results.
In Table 1, the contents and uniformities of the respective components were favorable for all samples.

**[Table 1]**

| | Na | K | Ca | Mg | Citric acid | Cl | Glucose |
|---|---|---|---|---|---|---|---|
| Example 1 | 100.2 ±0.22 | 99.5 ±0.21 | 99.6 ±0.47 | 98.1 ±0.36 | 98.3 ±0.25 | 101.2 ±0.12 | - |
| Example 2 | 101.7 ±0.12 | 98.3 ±0.33 | 98.4 ±0.41 | 99.1 ±0.35 | 99.1 ±0.36 | 101.1 ±0.35 | - |
| Example 3 | 101.6 ±0.43 | 100.3 ±0.61 | 99.4 ±0.25 | 98.6 ±0.32 | 98.7 ±0.38 | 101.2 ±0.35 | - |
| Example 4 | 100.1 ±0.15 | 99.6 ±0.52 | 100.1 ±0.27 | 99.1 ±0.13 | 99.6 ±0.41 | 100.9 ±0.42 | 98.7 ±0.67 |
| Example 5 | 100.3 ±0.16 | 98.6 ±0.23 | 100.4 ±0.31 | 99.5 ±0.38 | 98.4 ±0.25 | 100.8 ±0.26 | 99.4 ±0.71 |
| Example 6 | 101.0 ±0.35 | 99.4 ±0.37 | 99.6 ±0.51 | 98.9 ±0.61 | 99.2 ±0.22 | 100.9 ±0.25 | - |
| Example 7 | 100.9 ±0.24 | 99.4 ±0.41 | 99.2 ±0.46 | 98.7 ±0.54 | 99.4 ±0.41 | 101.1 ±0.28 | - |
| Example 8 | 101.2 ±0.34 | 99.6 ±0.62 | 99.2 ±0.48 | 99.1 ±0.51 | 98.8 ±0.35 | 100.7 ±0.31 | - |
| mean ± CV (%) | | | | | | | |

### (Test Example 2)

From each of the solid pharmaceutical preparations (A) of Example 1, Example 2, and Examples 5 to 8, 50 g thereof was sampled and then filled in an aluminum packaging container. 15 samples for stability test per example were prepared. The samples for the stability test were stored under the conditions of a temperature of 40°C and humidity of 75%. The aspects and contents of the components of the solid pharmaceutical preparations in the respective samples for stability test were measured at the onset of the storage and one month from the onset. Table 2 shows the measurement results of the aspects and contents. As is evident from the results in Table 2, all the pharmaceutical preparations of Examples 1, 2, and 5 to 8 retained their stabilities for one month under the conditions of a temperature of 40°C and humidity of 75%.

**[Table 2]**

| | | | Onset | After one month |
|---|---|---|---|---|
| Example 1 | Aspect | | White powder | White powder |
| | Contents | Na | 100.2±0.22 | 100.5±0.15 |
| | | K | 99.5±0.21 | 99.1±0.32 |
| | | Ca | 99.6±0.47 | 99.4±0.42 |
| | | Mg | 98.1±0.36 | 99.1±0.36 |
| | | Citric acid | 99.3±0.39 | 100.2±0.46 |
| | | Cl | 101.2±0.12 | 100.9±0.41 |
| | | Glu | - | - |
| Example 2 | Aspect | | White powder | White powder |
| | Contents | Na | 101.7±0.12 | 101.1±0.27 |
| | | K | 98.3±0.33 | 99.2±0.2.6 |
| | | Ca | 98.4±0.41 | 98.9±0.35 |
| | | Mg | 99.1±0.35 | 99.6±0.46 |
| | | Citric acid | 99.1±0.36 | 99.6±0.43 |
| | | Cl | 101.1±0.35 | 100.9±0.24 |
| | | Glu | - | - |
| Example 5 | Aspect | | White powder | White powder |
| | Contents | Na | 100.3±0.16 | 101.2±0.31 |
| | | K | 98.6±0.23 | 100.5±0.28 |
| | | Ca | 100.4±0.31 | 100.1±0.31 |
| | | Mg | 99.5±0.38 | 99.9±0.37 |
| | | Citric acid | 98.4±0.25 | 98.9±0.41 |
| | | Cl | 100.8±0.26 | 101.3±0.53 |
| | | Glu | 99.4±0.71 | 99.1±0.62 |
| Example 6 | Aspect | | White powder | White powder |
| | Contents | Na | 101.0±0.35 | 101.2±0.23 |
| | | K | 99.4±0.37 | 99.1±0.34 |
| | | Ca | 99.6±0.51 | 99.5±0.52 |
| | | Mg | 98.9±0.61 | 99.3±0.51 |
| | | Citric acid | 99.2±0.22 | 99.6±0.35 |
| | | Cl | 100.9±0.26 | 100.2±0.36 |
| Example 7 | Aspect | | White powder | White powder |
| | Contents | Na | 100.9±0.24 | 100.1±0.35 |
| | | K | 99.4±0.41 | 99.3±0.41 |
| | | Ca | 99.2±0.46 | 99.5±0.44 |
| | | Mg | 98.7±0.54 | 99.1±0.41 |
| | | Citric acid | 99.4±0.41 | 98.6±0.42 |
| | | Cl | 101.1±0.28 | 101.4±0.37 |
| Example 8 | Aspect | | White powder | White powder |
| | Contents | Na | 101.2±0.34 | 101.2±0.41 |
| | | K | 99.6±0.62 | 100.1±0.56 |
| | | Ca | 99.2±0.48 | 99.3±0.34 |
| | | Mg | 99.1±0.51 | 98.9±0.35 |
| | | Citric acid | 98.8±0.35 | 99.3±0.52 |
| | | Cl | 100.7±0.31 | 100.9±0.34 |

### Example 9

An aqueous solution was prepared by completely dissolving 1,000 parts by weight of sodium chloride, 24.4 parts by weight of potassium chloride, 30.1 parts by weight of calcium chloride, 16. 6 parts by weight of magnesium chloride, and 83. 9 parts by weight of citric acid in 2 parts by weight of purified water. Spray-drying granulation using the above-mentioned aqueous solution was carried out in a spray-drying granulator (New Speed Dryer, Type STREA-1, manufactured by Powrex, Co., Ltd.) under conditions of an intake-air temperature of 120°C and an exhaust temperature of 80°C. As a result, granular particles having an average particle size of about 500 µm were obtained. The granular particles are packaged in aluminum container, thereby providing a solid pharmaceutical preparation (A).
Furthermore, with respect to 1, 155 parts by weight of the solid pharmaceutical preparation (A), 385 parts by weight of sodium hydrogencarbonate was packaged in aluminum container and then provided as a solid pharmaceutical preparation (B), thereby obtaining a solid pharmaceutical preparation for dialysis composed of the solid pharmaceutical preparation (A) and the solid pharmaceutical preparation (B).

### Example 10

An aqueous solution was prepared by completely dissolving 1,000 parts by weight of sodium chloride, 24.4 parts by weight of potassium chloride, 30.1 parts by weight of calcium chloride, 16. 6 parts by weight of magnesium chloride, and 26.2 parts by weight of citric acid in 2, 625 parts by weight of purified water. Spray-drying granulation using the above-mentioned aqueous solution was carried out in a spray-drying granulator (New Speed Dryer, Type STREA-1, manufactured by Powrex, Co., Ltd.) under conditions of an intake-air temperature of 120°C and an exhaust temperature of 80°C. As a result, granular particles having an average particle size of about 500 µm were obtained. 1097.3 parts by weight of the granular particles was mixed with 88.3 parts by weight of sodium citrate in a V-type mixer for about 10 minutes, and the granular mixture thus obtained was then packaged in aluminum container to provide a solid pharmaceutical preparation (A).
Furthermore, with respect to 1, 186 parts by weight of the solid pharmaceutical preparation (A), 385 parts by weight of sodium hydrogencarbonate was packaged in aluminum container and then provided as a solid pharmaceutical preparation (B), thereby obtaining a solid pharmaceutical preparation for dialysis composed of the solid pharmaceutical preparation (A) and the solid pharmaceutical preparation (B).

### Example 11

As is the case with Example 9, granular particles were obtained (about 500 µm in average particle size).
In addition, 1, 000 g of 25 w/w% aqueous glucose solution was prepared. 1,000 g of glucose powders having an average particle size of 180 micrometer were placed in a rolling fluidized-bed granulator (MP-01, manufactured by Powrex, Co., Ltd.), and the glucose particles which were made to flow were then sprayed with 500 g of the aqueous glucose solution under conditions of an intake-air temperature of 60°C and a rotor speed of 300 rpm, thereby obtaining glucose particles having an average particle size of about 450 µm.
164 parts by weight of the glucose particles were added to 1,155 parts by weight of the above-mentioned granular particles, and then mixed in a V-type mixer for about 10 minutes, and the granular mixture thus obtained was packaged in aluminum container, thereby providing a solid pharmaceutical preparation (A).
Furthermore, with respect to 1, 314 parts by weight of the solid pharmaceutical preparation (A), 385 parts by weight of sodium hydrogencarbonate was packaged in aluminum container and then provided as a solid pharmaceutical preparation (B), thereby obtaining a solid pharmaceutical preparation for dialysis composed of the solid pharmaceutical preparation (A) and the solid pharmaceutical preparation (B).

### (Test Example 3)

From the solid pharmaceutical preparations (A) of the respective samples obtained in Examples 9 to 11 as described above, 10 g thereof were arbitrarily collected three times and each of them was then dissolved in water to prepare 50 mL of an aqueous solution. The contents of the respective components in the solution were measured. Table 1 shows the ratio (%) of each mean of the measured contents to the theoretical value, and its CV (%) (variation coefficient).
Note that, sodium and potassium were each measured by using a flame photometer, calcium and magnesium were each measured by using ion chromatography, citric acid was measured by using HPLC-UV, chloride was measured by a silver nitrate titration method, and glucose was measured by using a polarimeter.

**[Table 3]**

| | Na | K | Ca | Mg | Citric acid | Cl | Glucose |
|---|---|---|---|---|---|---|---|
| Example 9 | 101.1 ±0.31 | 99.8 ±0.35 | 99.3 ±0.42 | 99.3 ±0.37 | 99.6 ±0.36 | 100.4 ±0.27 | - |
| Example 10 | 100.9 ±0.22 | 99.6 ±0.37 | 98.8 ±0.42 | 98.6 ±0.26 | 100.4 ±0.46 | 100.8 ±0.19 | - |
| Example 11 | 100.8 ±0.29 | 99.3 ±0.45 | 99.1 ±0.35 | 100.3 ±0.45 | 99.2 ±0.48 | 100.2 ±0.21 | 98.7 ±0.49 |

| | | | | | | | |
|---|---|---|---|---|---|---|---|
| (mean ± CV %) | | | | | | | |

Table 3 reveals that the contents and uniformities of the respective components were good for all of the samples.

### (Test Example 4)

Each sample solid pharmaceutical preparations (A) of Examples 9 to 11 was packaged in aluminum container in a packaging amount of 50 g. Then, a stability test was conducted at 40°C, 75%RH and then the aspects and contents of the respective components were determined at each time point of the measurement.
Table 4 shows the results of the stability test for the powdery dialysis solution (aluminum container package).

**[Table 4]**

| | | | Onset | After one month |
|---|---|---|---|---|
| Example 9 | Aspect | | White powder | White powder |
| | Contents | Na | 101.1±0.31 | 101.3±0.33 |
| | | K | 99.8±0.35 | 99.1±0.30 |
| | | Ca | 99.3±0.42 | 99.4±0.39 |
| | | Mg | 99.3±0.37 | 100.3±0.29 |
| | | Citric acid | 99.6±0.36 | 99.1±0.41 |
| | | Cl | 100.4±0.27 | 100.1±0.35 |
| | | Glu | - | - |
| Example 10 | Aspect | | White powder | White powder |
| | Content s | Na | 100.9±0.22 | 101.3±0.25 |
| | | K | 99.6±0.37 | 99.3±0.31 |
| | | Ca | 98.8±0.42 | 99.4±0.51 |
| | | Mg | 98.6±0.26 | 99.1±0.26 |
| | | Citric acid | 100.4±0.46 | 100.6±0.23 |
| | | Cl | 100.8±0.19 | 101.3±0.34 |
| | | Glu | - | - |
| Example 11 | Aspect | | White powder | White powder |
| | Content s | Na | 100.8±0.29 | 101.1±0.38 |
| | | K | 99.3±0.45 | 99.5±0.29 |
| | | Ca | 99.1±0.35 | 100.1±0.42 |
| | | Mg | 100.3±0.45 | 100.3±0.41 |
| | | Citric acid | 99.2±0.48 | 99.4±0.44 |
| | | Cl | 100.2±0.21 | 101.3±0.29 |
| | | Glu | 98.7±0.49 | 99.4±0.52 |

### INDUSTRIAL APPLICABILITY

The solid pharmaceutical preparation for dialysis of the present invention can be dissolved in water before carrying out dialysis treatment to be provided as a dialysate for medical use.

## Claims

1. A solid pharmaceutical preparation for dialysis, comprising the following two solid pharmaceutical preparations:
a solid pharmaceutical preparation (A) having an average particle size of about 100 µm to 1,500 µm and containing one or more electrolytes selected from the group consisting of sodium chloride, calcium chloride, magnesium chloride, and potassium chloride and an organic acid other than acetic acid and/or a salt of the organic acid and
a solid pharmaceutical preparation (B) containing sodium bicarbonate.

2. The solid pharmaceutical preparation for dialysis according to claim 1, wherein the organic acid other than the acetic acid is at least one organic acid selected from the group consisting of citric acid, oxalic acid, tartaric acid, maleic acid, ascorbic acid, oxaloacetic acid, gluconic acid, isocitric acid, malic acid, and pyruvic acid.

3. The solid pharmaceutical preparation for dialysis according to claim 1, wherein:
the solid pharmaceutical preparation (A) comprises particles each core particles containing sodium chloride covered with a coating layer containing at least one electrolyte selected from the group consisting of calcium chloride, magnesium chloride, and potassium chloride, a plurality of particles being bound to each other.

4. A process for producing the solid pharmaceutical preparation for dialysis according to claim 1, comprising the steps of:
(1) spraying an aqueous solution containing at least one electrolyte selected from the group consisting of calcium chloride, magnesium chloride, and potassium chloride onto core particles containing sodium chloride, and then drying the core particles; and
(2) mixing the particles obtained by the step (1) with particles containing citric acid and/or a salt thereof, thereby producing a solid pharmaceutical preparation (A).

5. A process for producing the solidpharmaceutical preparation for dialysis according to claim 1, comprising the step of spraying an aqueous solution containing at least one electrolyte selected from the group consisting of calcium chloride, magnesium chloride, and potassium chloride, and an organic acid other than acetic acid and/or a salt thereof onto core particles containing sodium chloride, and then drying the core particles, thereby producing a solid pharmaceutical preparation (A).

6. Thesolid pharmaceuticalpreparationfor dialysis according to claim 1, wherein the solid pharmaceutical preparation (A) comprises:
first particles containing one or more electrolytes selected from the group consisting of sodium chloride, calcium chloride, magnesium chloride, and potassium chloride, the electrolytes being uniformly distributed within the particles; and
second particles containing an organic acid other than acetic acid and/or a salt of the organic acid.

7. The solid pharmaceutical preparation for dialysis according to claim 1, wherein the solid pharmaceutical preparation (A) comprises particles containing one or more electrolytes selected from the group consisting of sodium chloride, calcium chloride, magnesium chloride, and potassium chloride, and an organic acid other than acetic acid and/or a salt of the organic acid, the electrolytes and the organic acid other than acetic acid and/or the salt of the organic acid being uniformly distributed within the particles.

8. A process for producing the solid pharmaceutical preparation for dialysis according to claim 1, comprising the steps of:
(1) spraying and drying an aqueous solution containing at least one electrolyte selected from the group consisting of sodium chloride, calcium chloride, magnesium chloride, and potassium chloride to thereby obtain granulated substances; and
(2) mixing the granulated substances with particles containing citric acid and/or a salt thereof, thereby producing a solid pharmaceutical preparation (A).

9. A process for producing the solid pharmaceutical preparation for dialysis according to claim 1, comprising the step of spraying and drying an aqueous solution containing at least one electrolyte selected from the group consisting of sodium chloride, calcium chloride, magnesium chloride, and potassium chloride, and an organic acid other than acetic acid and/or a salt thereof, thereby producing a solid pharmaceutical preparation (A).
